# EUROPEAN PATENT SPECIFICATION

(11) **EP 0 719 571 B1**
(45) Date of publication and mention of the grant of the patent: **15.06.2005**
(21) Application number: 95120604.4
(22) Date of filing: 27.12.1995
(51) Int. Cl.: A61N 5/04, A61N 5/10

(54) **Medical apparatus**
Medizinische Vorrichtung
Appareillage médical

(30) Priority: 27.12.1994 JP 32584794; 27.12.1994 JP 32607794; 27.12.1994 JP 32607894; 16.03.1995 JP 5708895; 02.11.1995 JP 28609495
(43) Date of publication of application: 03.07.1996
(73) Proprietor: Olympus Optical Co., Ltd., Tokyo 151-0072 (JP)
(72) Inventor: Namioka, Yasuhiro, c/o Olympus Optical Co., Ltd., Hachioji-shi, Tokyo (JP); Hatta, Shinji, c/o Olympus Optical Co., Ltd., Hachioji-shi, Tokyo (JP); Inaba, Makoto, c/o Olympus Optical Co., Ltd., Hachioji-shi, Tokyo (JP); Nagase, Toru, c/o Olympus Optical Co., Ltd., Hachioji-shi, Tokyo (JP); Furukawa, Nobuyuki, c/o Olympus Optical Co., Ltd., Hachioji-shi, Tokyo (JP); Tagawa, Motoyuki, Olympus Optical Co., Ltd., Hachioji-shi, Tokyo (JP); Tanaka, Hiroaki, c/o Olympus Optical Co., Ltd., Hachioji-shi, Tokyo (JP)
(74) Representative: Winter, Brandl, Fürniss, Hübner, Röss, Kaiser, Polte Partnerschaft Patent- und Rechtsanwaltskanzlei

(56) References cited:
- WO-A-92/10932
- DE-A- 4 209 844
- US-A- 3 280 816
- US-A- 4 292 960
- US-A- 4 989 601
- US-A- 5 106 360
- US-A- 5 364 336
- US-A- 5 531 662
- CHEUNG A Y,AL-ATRASH J: "Microwave hyperthermia for cancer therapy" IEE PROC. A,PHYS. SCI. MEAS. INSTRUM. MANAGE. EDUC. REV. (UK), vol. 134, June 1987, 493-522, pages 493-522, XP002044563
- HEISSRATH M,PRIEMER J,SCHORCHT J,ZIMMERMANN M: "utilization of ct image in the preparation for thermo-radiotherapy" BIOMED.TECH. (GERMANY),BIOMEDIZINISCHE TECHNIK, vol. 38, no. 9, September 1993, GERMANY, pages 213-216, XP002044564

## Description

The present invention relates to a medical apparatus for treating an affected part of a patient, such as cancer.

Thermotherapy has been known as a treatment on an affected part of a patient, such as cancer. The thermotherapy is effective because cancer cells are less resistant to heat than unaffected cells. In the thermotherapy, a high-frequency current is supplied to the affected part, thereby heating the affected part.

To supply the high-frequency current to the affected part of the patient, a surgeon uses a thermotherapeutic applicator having two electrodes. More specifically, the surgeon positions the applicator such that the electrodes clamp the affected part between them and supplies the high-frequency current from one electrode to the other through the affected part.

Another type of therapy, known as radiotherapy, is also applied to treat cancer cells, and a medical apparatus for use in radiotherapy, known as radiotherapeutic apparatus, is used in practice. This apparatus has a radiation source, from which radiation is applied onto from the source onto the cancer cells to destroy the cancer cells.

Medical researches conducted recently have shown that a patient's affected part can be treated more effectively by conducting thermotherapy and radiotherapy simultaneously than by carrying out one of these these types of therapies.

No matter how quickly the two therapies are conducted one after the other, there still remains a time lag between the these therapies. Consequently, the affected part cannot be treated with a sufficiently high efficiency.

US 5,106,360 discloses a thermotherapeutic apparatus comprising an internal applicator adapted to be inserted into a body cavity of a patient. The applicator includes an electrode for warming the tissues of the patient's body and an insertion passage for inserting a radiation source used in radiotherapy by means of a push rod, so that the radiation source can be placed in the vicinity of the electrode before or during thermotherapy.

In WO 92/10932 is disclosed a thermotherapeutic probe for radiating microwave and nuclear radiation. Into an interstitial or intracavity probe a microwave radiator is incorporated, wherein this radiator is formed of a radioactive substance and adapted to simultaneously deliver microwave energy and nuclear radiation to living tissue.

Further, an apparatus and a method for application of radioactive and microwave energy to the body is described in US 4,292,960. The applicator has two ovoids and a tandem and is positionable into the vagina such that the ovoids are placed at the walls of the cervix and the tandem is inserted into the uterine cavity. In the tandem a pellet tube loaded with Caesium-137 pellets can be inserted in a hollow shaft. The tandem further comprises two additional hollow shafts for inserting two coaxial cables with unshielded ends so that a microwave signal can be launched from these unshielded ends. Also in the ovoids can be loaded Cesium-137 pellets and can be inserted a coaxial cable with an unshielded end to apply radioactive and microwave energy.

A radio-thermo-therapy applicator is disclosed in DE 42 09 844 A1 which provides simultaneous application of radioactive rays and heat. The radioactive source is incorporated in a cooled applicator part inserted in a body cavity, via an attached handpiece. The radioactive source is interchangeable with an activator for supplying HF energy to the introduced applicator part for heating the tumour tissue. In case the activator itself is hollow, the radioactive source can be inserted into the inside of the applicator.

It is the object of the present invention to provide an improved medical apparatus with which a surgeon can perform both radiotherapy and thermotherapy at the same time on an affected part of a patient, thereby to treat the affected part with improved efficiency.

To attain this object, there is provided a medical apparatus according to claim 1.

By using this medical apparatus, a surgeon can conduct radiotherapy and thermotherapy simultaneously, treating an affected part of a patient effectively.

This invention can be more fully understood from the following detailed description when taken in conjunction with the accompanying drawings, in which:
Fig. 1 is a partially sectional view of the intra-cavity applicator incorporated in a medical apparatus according to a first comparative example;
Fig. 2A is a transverse sectional view of the shaft of the intra-cavity applicator shown in FIG. 1;
Fig. 2B is a transverse sectional view of the shaft of a modified intra-cavity applicator for use in the first comparative example;
Fig. 3 is a schematic diagram showing the medical apparatus according to the first comparative example, explaining how the apparatus is used to conduct thermotherapy and radiotherapy at the same time;
Fig. 4A shows the intra-cavity applicator inserted in the esophagus of a patient;
Fig. 4B illustrates the intra-cavity applicator inserted in the rectum and colon of a patient;
Fig. 4C shows the intra-cavity applicator inserted in the bile duct of a patient;
Fig. 4D depicts the intra-cavity applicator inserted in the trachea and bronchus of a patient;
Fig. 4E shows the intra-cavity applicator inserted in the prostate and bladder of a patient;
Fig. 5 illustrates the intra-cavity applicator inserted in the vagina and uterus of a patient;
Fig. 6A is a perspective view showing the shaft of the intra-cavity applicator incorporated in a medical apparatus which is a second comparative example;
Fig. 6B is a perspective view showing the proximal end portion of the applicator shown in FIG. 6A;
Fig. 7A is a perspective view of the intra-cavity applicator incorporated in a medical apparatus which is a third comparative example;
Fig. 7B is a sectional view of the applicator shown in FIG. 7A, taken along line 7B-7B;
Fig. 8 is a perspective view showing the intra-cavity applicator used in a medical apparatus according to a fourth comparative example;
Fig. 9 is a plan view of the intra-cavity applicator used in a medical apparatus according to a first embodiment of the invention;
Fig. 10A is a perspective view of the distal end portion of the intra-cavity applicator incorporated in a medical apparatus which is a second embodiment of this invention;
Fig. 10B is a perspective view of the proximal end portion of the intra-cavity applicator illustrated in FIG. 10A;
Fig. 11A is a plan view showing the shaft of the intra-cavity applicator used in a medical apparatus according to a third embodiment of the present invention;
Fig. 11B is a plan view of the proximal end portion of the applicator illustrated in FIG. 11A;
Fig. 11C is a front view showing the proximal end portion of the applicator shown in FIG. 11A;
Fig. 11D is a plan view of the graduation provided on the shaft of the applicator shown in FIG. 11A;
Fig. 12 is a partially sectional view of the thermotherapeutic applicator incorporated in a medical apparatus according to a fifth comparative example;
Fig. 13 is a cross-sectional view of the applicator, taken along line 52-52 shown in FIG. 12;
Fig. 14A is a diagram explaining how radiation is distributed by the applicator shown in FIG. 12;
Fig. 14B is a diagram representing how radiation is distributed in a plane perpendicular to the axis of the applicator shown in FIG. 12, when radiation sources are set in all radiation source passages of the applicator;
Fig. 14C is a diagram showing how radiation is distributed in a plane perpendicular to the axis of the applicator shown in FIG. 12, when a radiation source is set in only one of the radiation source passages;
Fig. 14D is a diagram showing how radiation is distributed in a plane perpendicular to the axis of the applicator shown in FIG. 12, when radiation sources set in the adjacent three of the radiation source passages;
Fig. 14E is a diagram showing how radiation is distributed when radiation sources are set in two of the radiation source passages and displaced in the axial direction of the applicator shown in FIG. 12;
Fig. 15 is a cross-sectional view the thermotherapeutic applicator incorporated in a medical apparatus according to a fourth embodiment of the present invention;
Fig. 16 is a diagram representing how radiation is distributed in a plane perpendicular to the axis of the applicator shown in FIG. 15, when radiation sources set in the adjacent three of the radiation source passages;
Fig. 17 is a perspective view of the thermotherapeutic applicator of the fourth embodiment;

Embodiments of the present invention will be described with reference to the accompanying drawings.

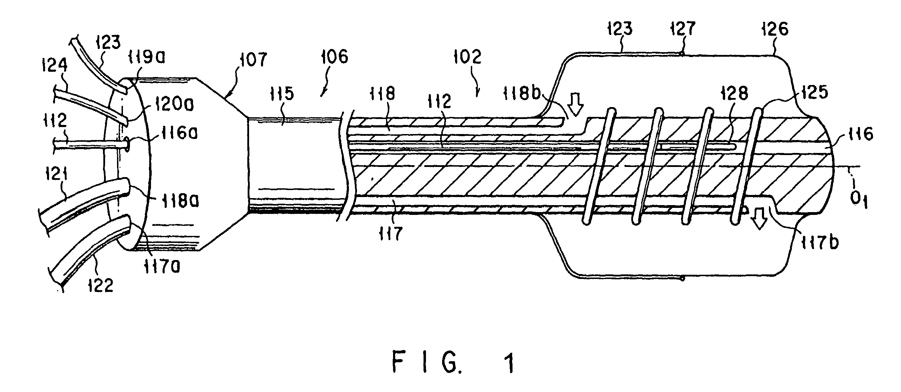

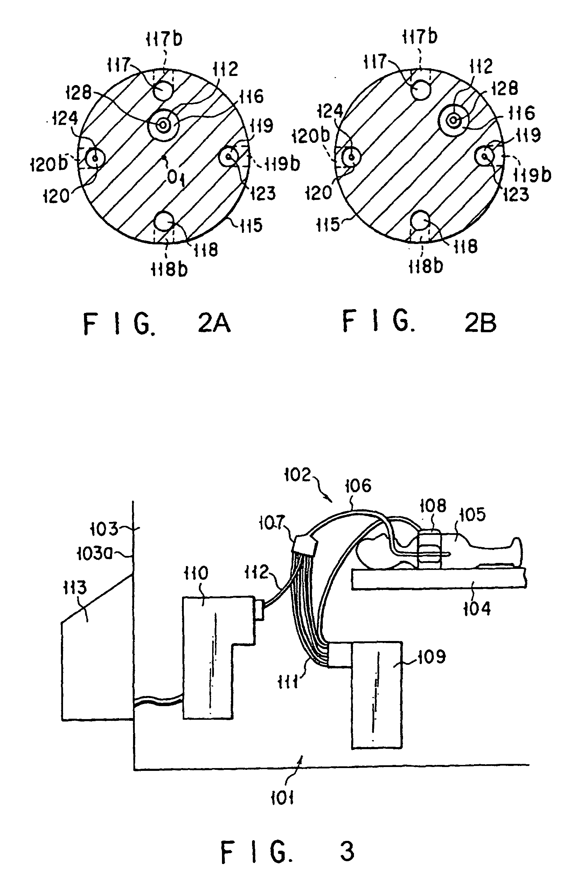

A medical apparatus as a first comparative example will be described, with reference to Figs. 1,2A,2B,3,4A to 4E and 5. Fig. 3 is a schematic representation of this medical apparatus 101 which is installed in an operating room 103. As seen from FIG. 3, the room 103 is electromagnetically shielded from any other room by radiation-shielded walls 103a. Installed also in the operating room 103 is a bed or an operating table 104, on which a patient 105 lies.

As shown in FIG. 3, the medical apparatus 101 comprises an intra-cavity applicator 102, an extracorporeal applicator 108, a hyperthermia device 109, a radiotherapeutic device 110, and a radiotherapy controller 113. The applicators 108 and 109, both fixed to the patient 105, are connected to the hyperthermia device 109 by cables and tubes 111. A radiation source tube 112 is connected at its proximal end to the radiotherapeutic device 110. The radiotherapy controller 113 is provided outside the operating room 103 and connected to the radiotherapeutic device 110.

The intra-cavity applicator 102 comprises an insertion section 106 and a proximal section 107. The insertion section 106 can be inserted into a body cavity of the patient 102. The proximal section 107 is coupled to the proximal end of the insertion section 106.

FIG. 1 shows the major components of the intra-cavity applicator 102. The insertion section 106 of the applicator 102 comprises a flexible shaft 115. As shown in FIG. 2A, the shaft 115 has a radiation source passage 116 which extends parallel to the axis O₁. The shaft 115 has a multi-lumen structure. Four parallel lumina 117 to 120 are provided in the circumferential surface of the shaft 115 and extend parallel to the axis O₁ thereof. The four lumina 117 to 120 are used as a cooling water supplying passage 117, a cooling water discharging passage 118, a temperature sensor guiding hole 119, and an electrode cable passage 120.

The lumina 117 to 120 need not take the positions specified in FIG. 2A. Rather, they may be arranged at other positions, as long as each has ends opening at the distal and proximal ends of the shaft 115, respectively. Likewise, the radiation source passage 116 need not assume the position shown in FIG. 18A; it may take a different position, so long as it extends parallel to the axis O₁ of the shaft 115.

The proximal end of the intra-cavity applicator 102 has five openings 116a to 120a, which are the proximal ends of the radiation source passage 116, cooling water supplying passage 117, cooling water discharging passage 118, temperature sensor guiding hole 119, and electrode cable passage 120. A water supplying tube 121 is connected at the distal end to the opening 117a of the cooling water supplying passage 117. A water discharging tube 122 is connected at the distal end to the opening 118a of the cooling water discharging passage 118. A temperature sensor cable 123 is inserted into the opening 119a, an electrode cable 124 is inserted into the opening 120a, and a radiation source guiding tube 112 is inserted into the opening 116a.

As illustrated in FIG. 1, a high-frequency electrode 125 is coiled around the distal end portion of the shaft 115. A balloon 126 is mounted on the distal end portion of the shaft 115, covering the high-frequency electrode 125. The electrode 125 is not limited to a coiled one. It may be replaced by a mesh electrode or a plate-like electrode.

The balloon 126 is made of an elastic sheet and secured at its front end to the distal end portion of the shaft 115 , and at its rear end to the proximal end portion of the shaft 115. The distal end portion of the shaft 115 is located in front of the high-frequency electrode 125, and the proximal end portion thereof is located at the back of the electrode 125.

The intra-cavity applicator 102 has four openings 117b to 120b in the circumferential surface of its distal end portion. The openings 117b to 120b are the proximal ends of the cooling water supplying passage 117, cooling water discharging passage 118, temperature sensor guiding hole 119, and electrode cable passage 120. Of these openings, the openings 117b, 118b and 120b open to the interior of the balloon 126. The cooling water is supplied into the balloon 126 through the cooling water supplying passage 117 and discharged from the balloon 126 through the cooling water discharging passage 118. Thus, the cooling water is circulated in the balloon 126.

An electrode cable 124 extends through the electrode cable passage 120. The distal end portion of the cable 124 projects into the balloon 126 from the opening 120b of the electrode cable passage 120. The electrode cable 124 is connected at its distal end to the high-frequency electrode 125.

The opening 119a of the temperature sensor guiding hole 119 opens to the exterior of the balloon 126. A temperature sensor cable 123 extends through the hole 119 and is connected at its distal end to a temperature sensor 127. The distal end portion of the cable 123 protrudes through the opening 119a from the shaft 115, at a position in front the balloon 126. The temperature sensor 127 is mounted on the balloon 126; it is, for example, a thermocouple, a temperature sensor having a platinum element, an optical-fiber temperature sensor, a thermistor, or the like.

Inserted in the radiation source passage 116 of the shaft 115 is a radiation source tube 112 which contains a radiation source 128. As shown in FIG. 1, the radiation source 128 is located in the middle part of that heating section of the shaft 115, around which the high-frequency electrode 125 is wound.

The radiation source passage 116 need not extend to the distal end of the shaft 115. Rather, it may extends only to a position where the radiation source 128 is located to oppose the balloon 126. In this case, the shaft 115 has no opening at its distal end at all. The the radiation source 128 is automatically set at that position when the radiation source tube 112 is inserted into the passage 116 until its distal end abuts on the closed end of the passage 116. Since the radiation source passage 116 extends not along the axis O₁ of the shaft 115 but parallel thereto, the radiation source 128 can be located near the tumor, when moved along the circumferential surface of the shaft 115 by rotating the shaft 115.

Hence, sufficiently intense radiation can be applied to the tumor.

It will be explained how the medical apparatus according to the fourteenth embodiment is used in the operating room 103 which is electromagnetically shielded from any other room by radiation-shielded walls 103a as shown in FIG. 3. At first, the water supplying passage 117 and the water discharging tube 122 are evacuated. The cooling water is thereby drawn from the balloon 11, deflating the balloon 126. Then, the insertion section 106 of the intra-cavity applicator 102 is inserted into the body cavity of the patient 105. The radiation source tube 112 is inserted into the passage 116, either before or after the insertion section 106 is inserted into the body cavity.

After the insertion section 106 is inserted into the body cavity, cooling water is supplied into and circulated in the balloon 126. The balloon 126 is thereby inflated and comes into contact with the inner surface of the body cavity. As a result of this, the distal end portion of the intra-cavity applicator 102 is held steadfastly in the body cavity.

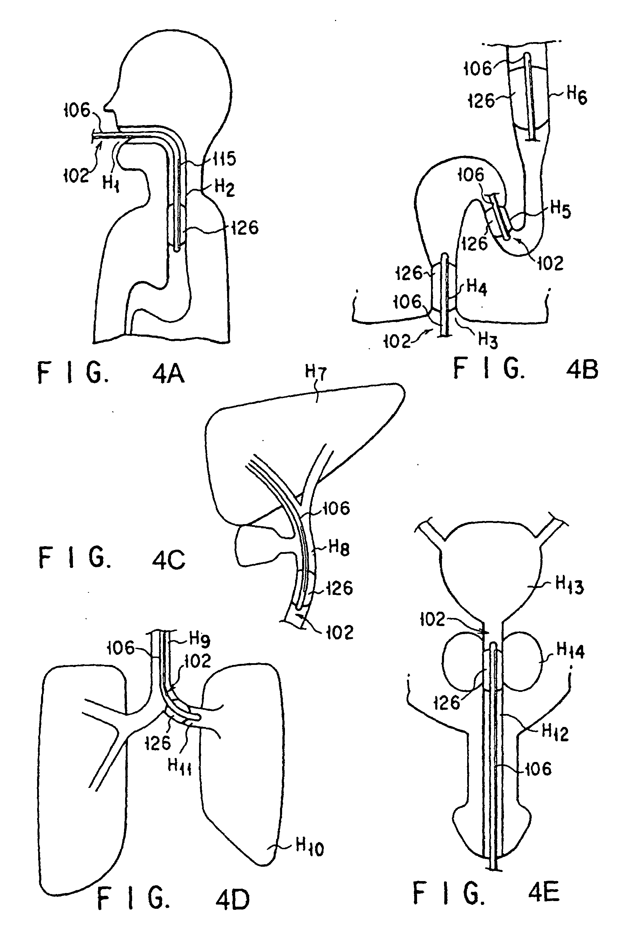

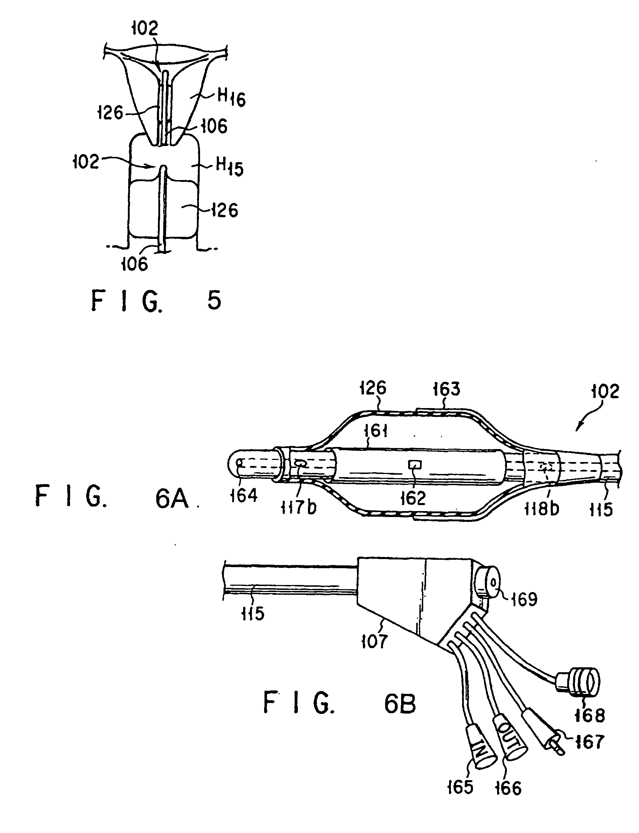

The intra-cavity applicator 102 can be inserted into the esophagus H₂ of a patient via the mouth H₁, as is shown in FIG. 4A. The applicator 102 may be inserted into the S-colon H₅ or the colon H₆ through the rectum H4, as is illustrated in FIG. 4B. The applicator 102 may be inserted into the bile duct H₈ of a patient, which is located in the vicinity of the lever H₇, as is illustrated in FIG. 4C. Further, the applicator 102 may be inserted into the trachea H₉ and bronchus H₁₁ of a patient both located near the lung H₁₀, as is shown in FIG. 4D. Still further, the applicator 102 may be inserted into the bladder H₁₃ and prostate H₁₄ through the urethra H₁₂, as is illustrated in FIG. 4E. Moreover, the applicator 102 may be inserted into the uterus H₁₆ through the vagina H₁₅, as is shown in FIG. 5.

The insertion section 106 of the intra-cavity applicator 102 is inserted into the body cavity and held at the affected part to be treated. The applicator 102 is then used to conduct thermotherapy and radiotherapy on the affected part. The order of the therapy steps differs in accordance with the protocol chosen by the surgeon who uses the medical apparatus 101.

It will explained how the apparatus 101 is used to perform high-dose radiotherapy and hyperthermia at the same time.

First, a high-frequency current is supplied to the high-frequency electrode 125, thereby initiating hyperthermia. When the temperature of hyperthermia becomes stable, the surgeon steps out of the operating room 103 which is electromagnetically shielded. Although the surgeon stays outside the operating room 103, the hyperthermia device 109 can continue the thermotherapy by hyperthermia reliably, only if device 109 can automatically control the hyperthermia temperature. The hyperthermia device 109 is positioned such that the surgeon can see the hyperthermia temperature displayed on the display panel on the device 109. It is desirable that a monitor be provided outside the room 103 to display the changes in hyperthermia temperature.

Next, the surgeon begins to perform radiotherapy. More precisely, he or she manipulates the radiotherapy controller 113 which is provided outside the operating room 103 and which is connected to the radiotherapeutic device 110. The radiation source 128 is introduced into the body cavity of the patient 105 from the device 110 through the radiation source tube 112. The source 128 is located in the distal end of the shaft 115 of the applicator, off the axis O₁ of the shaft 115 as illustrated in FIG. 1. This is because the tube 112 extends parallel to the axis O₁ of the shaft 115 of the applicator 102, not along the axis O₁.

The radiation source 128 emits radiation, whereby the affected part present in the body cavity is subjected to radiotherapy for a prescribed time. Upon completion of the radiotherapy, the surgeon operates the radiotherapy controller 113, moving the radiation source 128 back into the radiotherapeutic device 110. Even after the radiotherapy, the hyperthermia device 109 keeps on operating, whereby the hyperthermia is continued. The hyperthermia is automatically stopped upon lapse of a predetermined time.

If the source 128 emits a low dose of radiation, the radiation needs to be applied onto the affected part for a longer period of time. In this case, the surgeon selects a protocol of starting and stopping the hyperthermia device 109 during the radiotherapy achieved by operating the radiotherapeutic device 110. Alternatively, the heating by means of the device 109 may be started while the device 110 is being operated to conduct the radiotherapy.

Furthermore, the surgeon may selects a protocol of starting the hyperthermia device 109 after the radiotherapeutic device 110 has completed the radiotherapy, or a protocol of starting the radiotherapeutic device 110 after the hyperthermia device 109 has completed the hyperthermia. In either protocol, the intra-cavity applicator 102 is employed.

The medical apparatus 101 described above is advantageous in several respects.

First, the apparatus 101 enables the surgeon to treat an affected part of a patient, such as cancer, with high efficiency, since the hyperthermia device 109 and the radiotherapeutic device 110 can be operated to perform hyperthermia and radiotherapy at the same time.

Secondly, the radiation emitted from the source 128 can be applied concentratedly onto an affected part present on the inner surface of a body cavity. This is because the source 128 is located off the axis O₁ of the shaft 115 and can be moved to a position near the affected part by rotating the shaft 115. Hence, the intra-cavity applicator 102 serves to accomplish effective radiotherapy, as well as thermotherapy.

Further, the surgeon needs to insert the applicator 102 into the body cavity, but only once, in order to perform both radiotherapy and hyperthermia in the body cavity, because the applicator 102 has the high-frequency electrode 125 and contains the radiation source 128.

In addition, since the radiation source 128 is surrounded by the coiled high-frequency electrode 125, it would not distort the temperature distribution which the hyperthermia achieves in the affected part.

Lastly, the applicator 102 can be disinfected easily, provided that the distal end of the radiation source passage 116 is closed by some means.

The radiation source passage 116 of the shaft 115 may be opened at its distal end. The shaft 115 of the applicator 102 can then be made more easily than in the case where the passage 116 is closed at its distal end. In this case, the passage 116 can serve not only to guide the radiation source 128, but also to guide a guide wire or the insertion section of an endoscope or to supply air.

FIGS. 6A and 6B show a second comparative example. More precisely, they show the intra-cavity applicator 102 incorporated in a medical apparatus which is the second comparative example.

The intra-cavity applicator 102 is similar to its counterpart of the first comparative example (FIGS. 1 to 5). As seen from FIG. 6A, a hollow cylindrical, high-frequency electrode 161 is mounted on the distal end portion of the shaft 115 of the intra-cavity applicator 102. The electrode 161 is made of tungsten and is therefore opaque to radiation. The hollow cylindrical electrode 161 has in its middle part an opening 162 which has a size of 1 cm². A balloon 126 is mounted on the shaft 115, covering the high-frequency electrode 161. Two temperature sensors 163, each comprised of a thermocouple, are mounted on the outer surface of the balloon 126. The shaft 115 is made of silicone and has a coaxial catheter passage 164.

The shaft 115 also has a water supplying passage 117, a water discharging passage 118, a temperature sensor passage 119, and an electrode cable passage 120. These passages open at the proximal end portion 107 of the intra-cavity applicator 102. As shown in FIG. 6B, a water inlet connector 165 is connected to the proximal end of the passage 117, a water outlet connector 166 to the proximal end of the passage 118, a sensor connector 167 to the cable inserted in the passage 119 and connected to the temperature sensors 163 mounted on the balloon 126, and an electrode cable connector 168 to the electrode cable provided in the passage 120. The catheter passage 164 opens at the proximal end portion 107, too. A catheter guide 169 is fastened to the distal end of the catheter passage 164.

It will be explained how the intra-cavity applicator 102 is used to treat an affected part present in the body cavity of a patient. At first, the applicator 102 is inserted into the body cavity, with the balloon 126 deflated completely. Then, the inlet connector 165 and the outlet connector 166 are connected to a water circulating unit (not shown). Water is supplied from the water circulating unit into the balloon 126 through the water inlet connector 165 and the water supplying passage 117. The balloon 126 is thereby inflated, touching the inner surface of the body cavity. As a result, the applicator 102 is held in place within the body cavity. Even after the balloon 126 has been fully inflated, water is continuously supplied into the balloon 126, and excessive water flows from the balloon 126 into the water circulating unit through the water discharging passage 118 and the outlet connector 166. Thus, the water keeps on circulating through the loop formed of the balloon 126, the passages 116 and 117, the connectors 165 and 166 and the water circulating unit.

Thermotherapy can performed on the affected part by supplying a high-frequency current between the high-frequency electrode 161 of the applicator 102 and the electrode (not shown) secured to an extracorporeal applicator 108 of the type shown in FIG. 3.

In order to conduct radiotherapy on the affected part, an RI catheter (not shown) which is a radiation source is inserted into the catheter passage 164 via the catheter guide 169. The RI catheter emits radiation which will be applied to the affected part. The radiation is applied from the applicator 102, exclusively through the opening 162 of the high-frequency electrode 161. This is because electrode 161 is made of tungsten which opaque to radiation. Hence, to apply the radiation onto the affected portion, the applicator 102 is rotated around its axis until the opening 162 comes to oppose the affected part. Thus, the radiation emitted from the source 128 can be applied concentratedly onto any affected part present on the inner surface of the body cavity, in the same manner as in the first comparative example. The intra-cavity applicator 102 can therefore enables a surgeon to perform both radiotherapy and thermotherapy effectively.

In addition, any part other than the affected part in the body cavity is not exposed to radiation since the radiation emitted from the source is applied outwards through only the opening 162 of the high-frequency electrode 161.

The high-frequency electrode 161, which is a hollow cylinder, may be replaced by a hollow prism or a plate. The opening 162 may be of any size and any shape so long as it can be made in the electrode 161. It may be rectangular, circular, elliptical, or polygonal. Further, the electrode 161 may have two or more openings, instead of only one. Still further, the material of the electrode 161 is not limited to tungsten; it may be made of lead, lead glass, or the like.

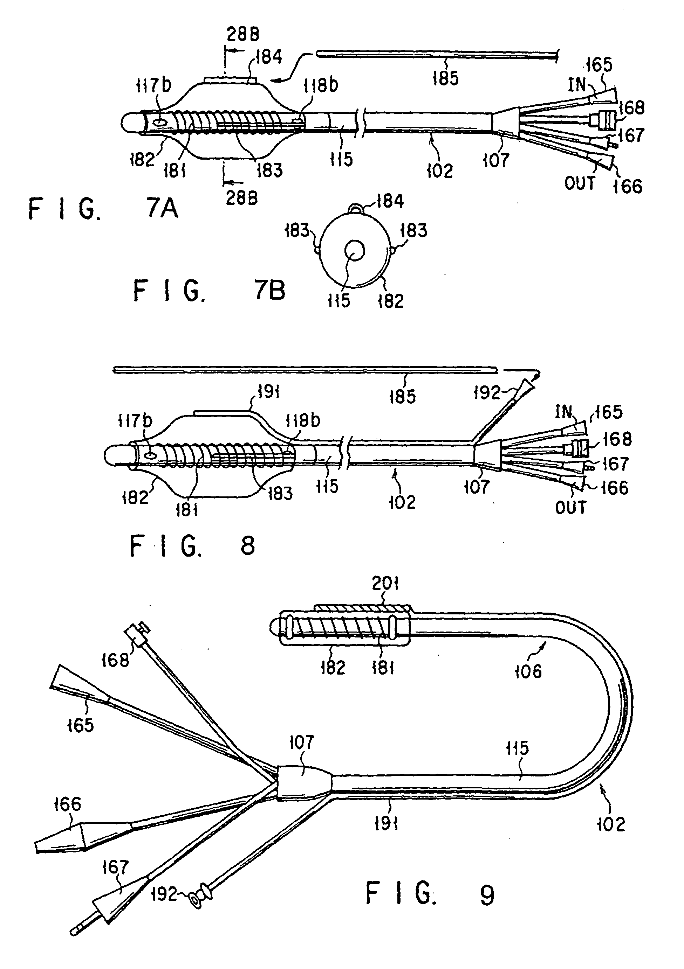

FIGS. 7A and 7B show a third comparative example, more precisely the intra-cavity applicator incorporated in a medical apparatus.

The intra-cavity applicator is similar to its counterpart of the second comparative example (FIGS. 6A and 6B) but somewhat different.

As seen from FIG. 7A, a high-frequency electrode 181 is coiled around the distal end portion of the intra-cavity applicator, and a balloon 182 is mounted on the distal end portion of the applicator, covering the entire electrode 181. Two temperature sensors 183, each comprised of a thermocouple, and a radiation source passage 184 are mounted on the outer surface of the balloon 182. The radiation source passage 184 is a silicone tube, into which a RI catheter 185 can be inserted. The passage 184 is closed at the distal end. Thus, the RI catheter 185 can be inserted into the passage 184 until it abuts on the closed distal end of the passage 184 and is thereby positioned.

As described above, the RI catheter 185 is inserted in the passage 184 mounted on the outer surface of the balloon 182. Therefore, the radiation emitted from the catheter 185 can be applied to an affected part in a body cavity, merely by rotating the shaft 115 around its axis O₁ to make the catheter 185 oppose the affected part. The radiation emitted from the RI catheter 185 can therefore be applied concentratedly onto any affected part present on the inner surface of the body cavity, as in the first comparative example. Hence, the intra-cavity applicator can enables a surgeon to perform both radiotherapy and thermotherapy with high efficiency.

In addition, neither the electrode 181 nor the balloon 182 would not affect the distribution of the radiation emitted from the catheter 185, because the RI catheter 185 is mounted on the outer surface of the balloon 182.

Moreover, it is easy to position the RI catheter 185 in the radiation source passage 184, only by inserting the catheter 185 into the passage 184 until the catheter 185 abuts on the closed distal end of the passage 184.

Sill further, the RI catheter 185 can be located very close to the inner surface of a body cavity as long as the balloon 182 remains in contact with the inner surface of the body cavity. This is because the radiation source passage 184 is mounted o the outer surface of the balloon 182. It is therefore possible to estimate the distance from the catheter 185 to the mucous membrane on the inner surface of the body cavity and to determine the distribution of radiation emitted from the catheter 185. As a result, the radiation can be accurately aimed at and concentratedly applied to the affected part.

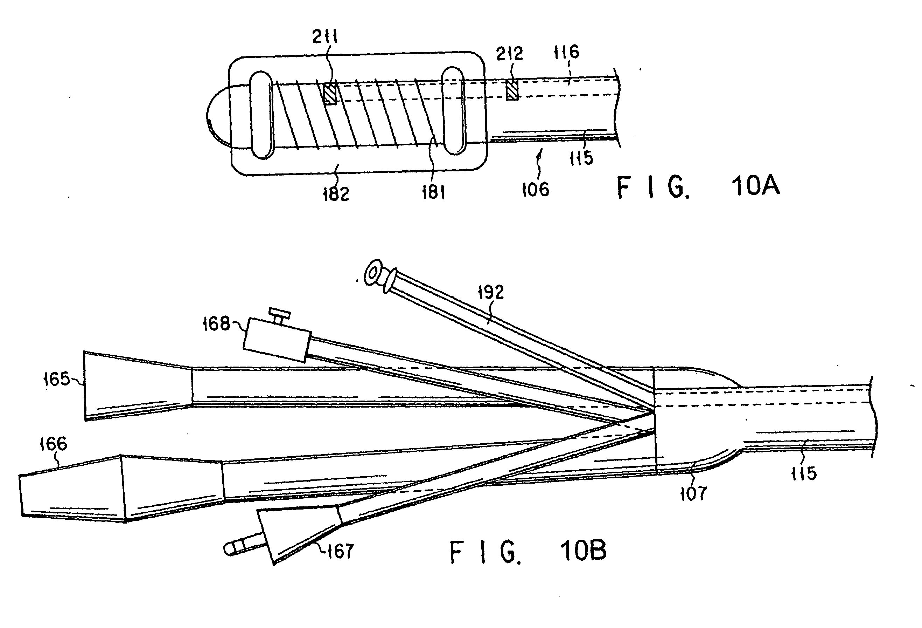

A medical apparatus according to the fourth comparative example will be described, with reference to FIG. 8 which shows the intra-cavity applicator 102 incorporated in the medical apparatus.

The intra-cavity applicator 102 is a modification of the applicator of the third comparative example (FIGS. 7A and 7B). As shown in FIG. 8, the applicator 102 is characterized in that an RI catheter tube 191 made of fluororesin such as Teflon is mounted partly on the shaft 115 and partly on the outer surface of the balloon 182. The tube 191 has its closed distal end located at the distal end of the shaft 115 and mounted on the balloon 183. The proximal end of the tube 191 is located at the proximal end portion 107 of the applicator 102 and connected to a catheter guide 192. An RI catheter 185 can be inserted into the tube 191 via the catheter guide 192, no matter whether the balloon 182 is inflated or deflated. The catheter 185 is inserted into the tube 191 until the catheter 185 abuts on the closed distal end of the tube 191, whereby the distal end of the catheter 185 is located on the balloon 182.

As mentioned above, the RI catheter tube 191 has its distal end portion mounted on the outer surface of the balloon 182, and the RI catheter 185 is inserted in the tube 191. Thus, to apply the radiation emitted from the catheter 185 to an effected part present in a body cavity, it suffices to rotate the shaft 115 around its axis O₁ to move the tube 191 to a position where the tube 191 faces the affected part.

The radiation emitted from the RI catheter 185 can therefore be applied concentratedly onto any affected part present on the inner surface of the body cavity, as in the first comparative example. Hence, the intra-cavity applicator can enables a surgeon to perform both radiotherapy and thermotherapy with high efficiency.

Moreover, the RI catheter 185 can be easily inserted into the RI catheter tube 191, and a radiation source can be inserted into the body cavity through the RI catheter tube 191.

Another medical apparatus which is the first embodiment of the invention will be described, with reference to FIG. 9 which shows the intra-cavity applicator 102 incorporated in the medical apparatus.

The intra-cavity applicator 102 is a modification of the applicator of the fourth comparative example (FIG. 8). As shown in FIG. 9, the applicator 102 is characterized in that an X-ray marker 201 opaque to X-rays is provided on the distal end portion of the RI catheter tube 191. To be more specific, the X-ray marker 201 is mounted on the outer surface of the balloon 182.

This intra-cavity applicator 102 is used in the following way to treat a tumor in a body cavity of a patient. A surgeon first inserts the applicator 102 into the body cavity. Then, the surgeon determines the position of the tumor in the cavity, from an X-ray image of the interior of the body cavity. From the X-ray image the surgeon can accurately determine the position of the distal end portion of the applicator 102 since the X-ray marker 201 is quite visible in the X-ray image. He or she rotates the shaft 115 of the applicator 102 around its axis, thereby positioning the distal end portion of the applicator 102 at the tumor, and inserts a RI catheter 185 into the RI catheter tube 191. Once the RI catheter 185 is set in the tube 191, the surgeon can perform both hyperthermia and radiotherapy on the tumor. The the RI catheter 185 may be inserted into the RI catheter tube 191 before the applicator 102 is inserted into the body cavity.

The RI catheter tube 191 has its distal end portion mounted on the outer surface of the balloon 182, and the RI catheter 185 is inserted in the tube 191, as in the fourth comparative example (FIG. 8). Thus, the radiation emitted from the catheter 185 can be applied to the tumor, merely by rotating the shaft 115 around its axis O₁ to make the tube 191 oppose the tumor.

The radiation emitted from the RI catheter 185 can be applied concentratedly onto any affected part present on the inner surface of the body cavity, as in the first comparative example (FIG. 1 to 5). Hence, the intra-cavity applicator 102 can enables a surgeon to perform both radiotherapy and thermotherapy with high efficiency.

In addition, the surgeon can accurately determines not only the position of the tumor but also the position of the distal end portion of the applicator 102 from an X-ray image of the body cavity. This is because the X-ray marker 201 provided on the distal end portion of the applicator 102 is clearly seen in the an X-ray image. It suffices for him or her to rotate the shaft 115 of the applicator 102 around its axis, to thereby position the distal end portion of the applicator 102 at the tumor. Once the distal end portion of the applicator 102 is so positioned, the radiation emitted from the RI catheter 185, which is inserted in the distal end portion of the applicator 102, is applied concentratedly onto the tumor.

A medical device according to the second embodiment of the present invention will be described with reference to FIGS. 10A and 10B. FIGS. 10A and 10B show the intra-cavity applicator 102 provided in this medical apparatus.

The intra-cavity applicator 102 shown in FIGS. 10A and 10B is a modification of the applicator 102 of the first comparative example (FIGS. 1 to 5). As shown in FIG. 10A, X-ray markers 211 and 212 are mounted on two distal end portions of the shaft 115. Both X-ray markers 211 and 212 are opaque to X rays. The first X-ray marker 211 indicates the position of the distal end of the radiation source passage 116 which is formed in the shaft 115 and which cannot be seen from outside. The second X-ray maker 212 indicates the position of a portion of the passage 116 which is proximal with respect to the distal end of the passage 116.

The intra-cavity applicator 102 is used in the following way to treat a tumor in a body cavity of a patient. At first, a surgeon inserts the applicator 102 into the body cavity. Then, he or she determines the position of the tumor in the cavity, from an X-ray image of the body cavity. The surgeon can see the images of both X-ray markers 211 and 212 in the X-ray image. The surgeon rotates the shaft 115 of the applicator 102 around its axis, until the images of the X-ray markers 211 and 212 move to the image of the tumor. As a result, the shaft 115 is positioned, with the distal end portion of the radiation source passage 116 placed at the tumor. The surgeon inserts a radiation source 128 into the radiation source passage 116. The surgeon can then perform hyperthermia and radiotherapy simultaneously on the tumor. The radiation source 128 may be inserted into the passage 116 before the shaft 115 is inserted into the body cavity.

The second embodiment can attain the same advantages as the first embodiment (FIG. 9). In addition, the X-ray image of the interior of the body cavity is clearer than in the case the applicator of the first embodiment is inserted into the body cavity. This is because the shaft 115 is coated with the X-ray markers over a smaller area than its counterpart of the first embodiment.

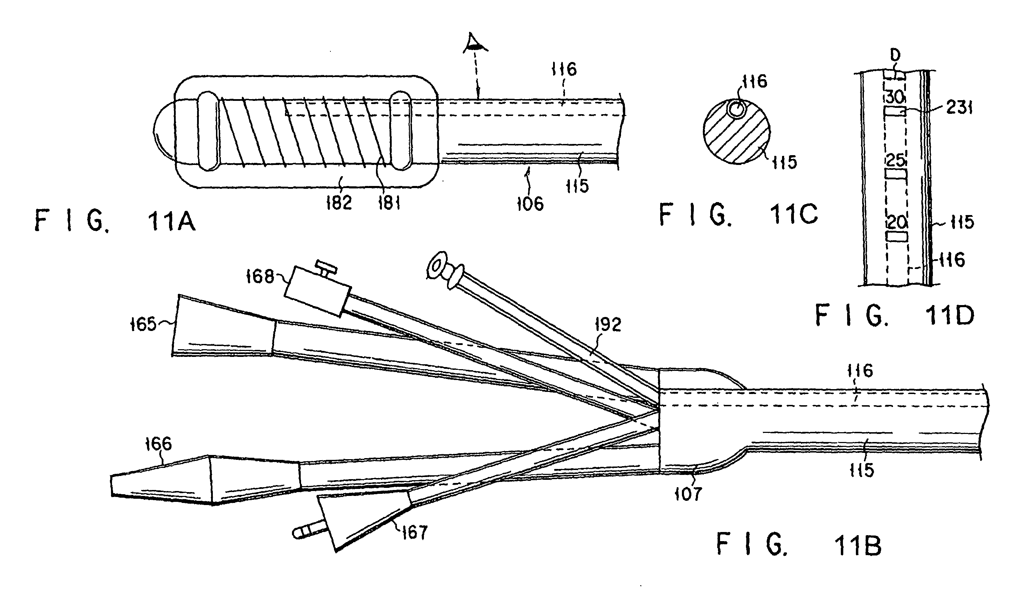

A medical apparatus according to the third embodiment of this invention will be described, with reference to FIGS. 11A to 11D which show an intra-cavity applicator.

The intra-cavity applicator of the third embodiment is a modification of the applicator 102 of the first comparative example (FIG. 1 to 5). It is characterized in that a graduation 231 is provided on that portion of the shaft 115 which opposes the radiation source passage 116 which is formed in the shaft 115 and which cannot be seen from outside. The radiation source passage 116 has a diameter D as shown in FIG. 11D.

Further, the graduation 231 is an X-ray marker which is opaque to X rays. A surgeon use the intra-cavity applicator in the following manner to treat a tumor in a body cavity of a patient. At first, he or she inserts the shaft 115 of the applicator into the body cavity and insert the radiation source into the radiation source passage 116. Then, the surgeon determines the position of the tumor and also the position of the graduation 231, from an X-ray image of the interior of the body cavity. He or she rotates the shaft 115 around its axis, until the graduation 231 move to the tumor. The surgeon can then perform hyperthermia and radiotherapy simultaneously on the tumor. The the radiation source may be inserted into the passage 116 after the shaft 115 is rotated to locate the graduation 231 at the tumor. Since the graduation 231 is an X-ray marker, the surgeon can place the radiation source at the desired position in the body cavity, within a shorter time than he or she can do so with a intra-cavity applicator having no X-ray marker.

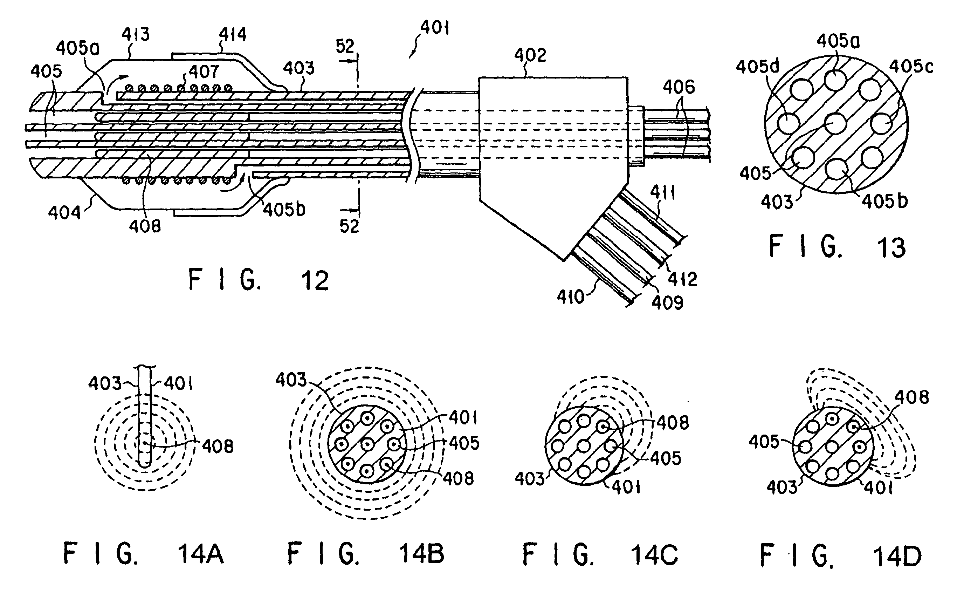

A medical device as a fifth comparative example will be described with reference to FIGS. 12 and 13 and FIGS. 14A to 14E. FIG. 12 shows the thermotherapeutic applicator 401 used in the medical apparatus. FIG. 13 is a cross-sectional view of the applicator 401. FIGS. 14A to 14E are diagrams explaining how radiation is distributed from the thermotherapeutic applicator 401.

As seen from FIG. 12, the thermotherapeutic applicator 401 comprises a proximal section 402, a shaft 403 and a distal section 404. The shaft 403 has multi-lumen structure. That is, as shown in FIG. 13, five radiation source passages 405, a water supplying passage 405a, a water discharging passage 405b, a temperature sensor passage 405c and an electrode cable passage 405d are formed in the shaft 403. The center radiation source passage 405 is coaxial with the shaft 403. The passages 405a, 405b, 405c and 405d extend parallel to the axis of the shaft 403. Four more radiation source passages 405 are formed in the shaft 403, extending parallel to the axis thereof and located among the passages 405a to 405d as shown in FIG. 13. Five radiation source tubes 406 are inserted into the radiation source passages 405, respectively.

A high-frequency electrode 407, which is a wire, is wound around the distal section 404 of the shaft 403. The electrode is used to conduct thermotherapy. A balloon 413 is fastened to the distal section 404, covering the high-frequency electrode 407.

Radiation sources 408 are inserted into the radiation source tubes 406 inserted in the radiation source passages 405. The sources 408 can be located in the passages 405, surrounded by the high-frequency electrode 407. As shown in FIG. 12, the radiation source passages 405 are opened at the distal end of the shaft 403. Nonetheless, the passages 405 may be closed at the distal end of the shaft 403.

At the proximal section 402, the water supplying passage 405a is connected to a water supplying tube 409, and the water discharging passage 405b to a water discharging tube 410. A temperature sensor cable 411 extends through the temperature sensor passage 405c, and an electrode cable 412 extends through the electrode cable passage 405d.

Cooling water can flow into the balloon 413 through the water supplying passage 405a and from the balloon 413 through the water discharging passage 405b, to circulate within the balloon 413. The temperature sensor cable 411 is led outside at the rear end of the balloon 413 and connected to a temperature sensor 414, which is mounted on the outer surface of the balloon 413. The electrode cable 412 is led out from the shaft 403 and connected to the electrode 407, within the balloon 413.

It will be explained how a surgeon use the thermotherapeutic applicator 401 to an affected part present in a patient's body cavity.

The applicator 401 is used an operating room of the type shown in FIG. 3, which is electromagnetically shielded. First, the surgeon connects the applicator 401 to a hyperthermia device (not shown) of the type shown in FIG. 3. He or she then evacuates the water supplying tube 409 and the water discharging tube 410. Cooling water is thereby drawn from the balloon 413, deflating the balloon 413. The surgeon inserts the applicator 401 into the patient's body cavity. The radiation source tube 406 is inserted into the passage 405, either before or after the insertion section of the applicator 401 is inserted into the body cavity.

After the insertion section of the applicator 401 is inserted into the body cavity, cooling water is supplied into and circulated in the balloon 413. The balloon 413 is thereby inflated and comes into contact with the inner surface of the body cavity. As a result, the distal end portion of the thermotherapeutic applicator 401 is held steadfastly in the body cavity.

Then, the surgeon starts performing thermotherapy. When the affected part in the body cavity is heated to a desired temperature, the surgeon steps out of the operating room. Outside the room, the surgeon operates a radiotherapy controller of the type shown in FIG. 3. The controller is connected to the radiotherapeutic device installed in the operating room. The radiation sources 408 are thereby inserted from the radiotherapeutic device into the radiation source tubes 406 which are inserted in the body cavity. The sources 408 are placed in the distal section 404 of the applicator 401 because the tubes 406 are inserted in the passages 405 of the applicator 401.

The radiation sources 408 set in the passages 405 emits radiation, which is applied onto the affected part present in the patient's body cavity. Radiotherapy is thus conducted on the affected part.

If the thermotherapeutic applicator 401 had only one radiation source passage 405, the radiation emitted from the applicator 401 would extend in all directions as shown in FIG. 14A, in a spherical space whose center is the radiation source 408 set in the thermotherapeutic applicator 401. The distribution of radiation emitted from the applicator 401 is determined by the position and size of the radiation source 408.

As mentioned above, the shaft 403 of the applicator 401 has five radiation source passages 405. The surgeon can inserts one to five radiation source tubes 406 into selected one or ones of the radiation source passages 405, in accordance with the shape, size and position of the affected part he or she is going to treat. The first radiation source passage 405 is coaxial with the shaft 403, whereas the remaining four source passages 405 are equidistant from the axis of the shaft 403 and spaced apart at regular intervals along the circumference of the shaft 403. Hence, when five radiation sources 408 are inserted in all radiation source passages 405, the radiation they emit is distributed uniformly in a circular plane which is perpendicular to the axis of the applicator 401 as is illustrated in FIG. 14B.

To apply radiotherapy on cancer developing on the entire inner surface of a body cavity, it is desirable that five radiation sources 408 be inserted in all radiation source passages 405 as shown in FIG. 14B. In this case, the radiation applied from the applicator 401 is five times as intense as in the case where only one radiation source 408 is set in the thermotherapeutic applicator 401.

Assume a radiation source 408 is inserted in one of the four radiation source passage 405 which extend parallel to the axis of the shaft 403, as shown in FIG. 14C. Then, the radiation emitted from the source 408 is distributed in a circular plane which is perpendicular to the axis of the applicator 401 and whose center is the source 408, not the axis of the shaft 403. This specific distribution of radiation is desirable to treat, for example, cancer developing on a limited region of the inner surface of the body cavity. So distributed, the radiation is applied to the cancer only. This makes effective radiotherapy possible.

Further assume that three radiation sources 408 are inserted in three adjacent ones of the radiation source passage 405 which extend parallel to the axis of the shaft 403, as shown in FIG. 14D. In this case, the radiation the sources 408 emit overlap and are distributed in the axial direction of the shaft 403. As this example teaches, radiation can be distributed in a way desirable for treating an affected part by inserting radiation sources 408 into selected ones of the radiation source passages 405.

To apply radiation to radiation onto cancer which extends along the applicator 401, two or more radiation sources 408 may be located in the radiation source passages 405 and displaced from one another in the axial direction of the applicator 401, as is shown in FIG. 14E. In this case, the radiation is distributed in an elliptical region which extends along the thermotherapeutic applicator 401. If the radiation thus distributed is less intense than desired, it suffices to insert more radiation sources 408 into the passages 405.

The surgeon needs to insert the applicator 401 into the body cavity, but only once, to perform thermotherapy and hyperthermia an affected part present in the body cavity. Thus, the affected part can be treated more easily than otherwise. Further, the use of the thermotherapeutic applicator 401 is advantageous in the following respects.

First, radiation sources 408 can be placed in any selected passage 405 and at any desired position along the axis thereof, because the shaft 403 has a plurality of radiation source passages 405. As a result, the radiation can be distributed in the following three. patterns:
(a) Radiation is distributed uniformly to the entire surface of a body cavity when radiation sources 408 are inserted in all radiation source passages 405 as shown in FIG. 14B. In this case, the radiation can be effectively applied to the cancer developing on the entire inner surface of the body cavity.
(b) Radiation is distributed to a limited region of the inner surface of the body cavity when radiation sources are inserted two or more of the radiation source passages as illustrated in FIG. 14C or 14D. The radiation can be applied to the cancer developing on that limited region of the inner surface of the body cavity, not to the normal tissues present on the other region of the inner surface.
(c) Radiation is distributed in an elliptical region extending along the applicator 401 when two or more radiation sources may be located in the radiation source passages and displaced from one another in the axial direction of the applicator 401, as is shown in FIG. 14E. The radiation can be applied to cancer which extends along the applicator 401.

Secondly, the dose of the radiation emitted from the applicator 401 can be increased, if necessary, merely by inserting more radiation sources 408 into the passages 405. Once the dose is thus increased, the time required to complete the radiotherapy can be shortened. (Generally, the amount of radiation a source emits decreases with time. If only one radiation source is used, it must keep emitting radiation until the total amount of radiation emitted becomes adequate.)

Thirdly, any radiation source 408 placed in the applicator 401 would not distort the temperature distribution which the hyperthermia achieves in the affected part. This is because the radiation source 408 is surrounded by the high-frequency electrode 407, Furthermore, the radiation source tubes 406 would not slips out of the applicator 401, provided that the radiation source passages 405 are closed at their distal ends. This ensures the safety of therapy. The passages 405, if opening at distal end, can be used for guiding an endoscope or an medical instrument into the body cavity, so that an affected part in the cavity may be observed and treated.

If a radiation source 408 is inserted into the first passage 405 coaxial with the shaft 403 of the applicator 401, the radiation it emits will be uniformly distributed in a circular plane whose center is on the axis of the shaft 403.

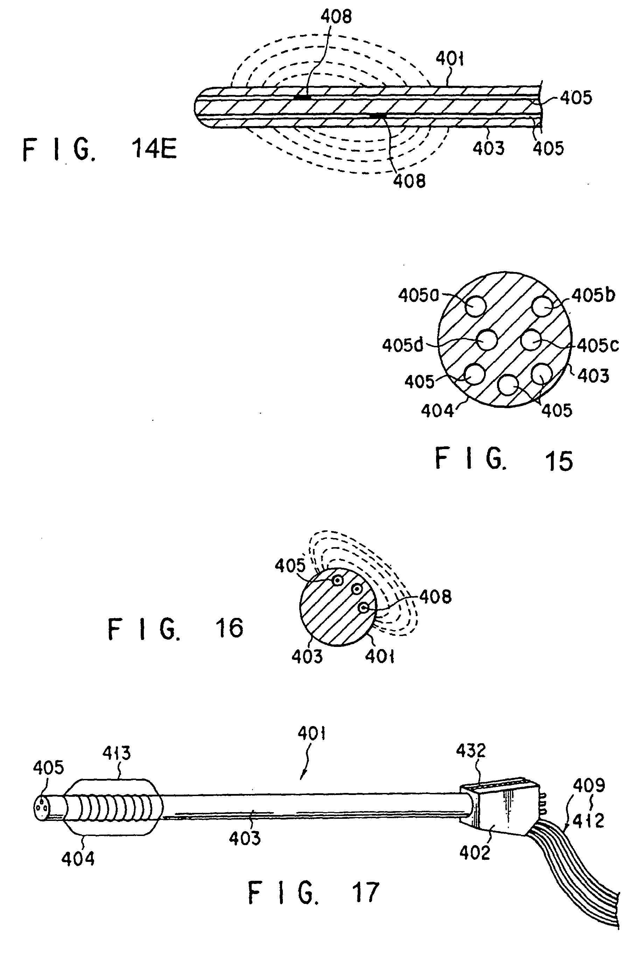

A medical apparatus according to the fourth embodiment of the present invention will be described, with reference to FIGS. 15 to 17. This embodiment differs from the fifth comparative example (FIGS. 12 and 13, FIGS. 14A to 14E) in the structure of the shaft 403 of the thermotherapeutic applicator 401.

As shown in FIG. 17, three radiation source passages 405 are provided side by side in a part of the near-surface region of the distal portion of the shaft 403. A mark 432 is provided on the proximal section 402 of the applicator 401, indicating the position of the radiation source passages 405.

The applicator 401 of the fourth embodiment is suitable for use in treating cancer developing in a limited region on the inner surface of a body cavity. To perform radiotherapy on such cancer, a surgeon inserts the applicator 401 into the body cavity. He or she then rotates the applicator 401 until the mark 432 comes to face the cancer. The radiation source passages 405 formed in the shaft 403 of the applicator 401 are thereby made to oppose the cancer. The positions the passages 405 take with respect to the cancer and the number of radiation sources 408 to be set in the passages 405 are changed in accordance with the size of the cancer. More specifically, if the cancer extends a short distance along the circumference of the body cavity, the sources 408 are used in small numbers to distribute radiation in a relatively small part of the inner surface of the body cavity. Conversely, it the cancer extends a long distance along the circumference of the cavity, the sources 408 are used in great numbers to distribute radiation in a comparatively large part of the inner surface of the body cavity.

Like the fifth comparative example, the fourth embodiment can apply radiation to the cancer developing on that limited region of the inner surface of the body cavity, not to the normal tissues present on the other region of the inner surface. Having less radiation source passages 405, the thermotherapeutic applicator 401 is mechanically stronger, can be made more easily, and can be manufactured at a lower cost, than its counterpart of the fifth comparative example.

## Claims

1. A medical apparatus for treating an affected part of a patient, comprising:
a thermotherapeutic means for conducting thermotherapy on the affected part said thermotherapeutic means comprising an intra-cavity thermotherapeutic applicator comprising a flexible shaft (115,403) defining an axis and a high-frequency electrode (181,407) and a balloon (182,413) both mounted to a distal end portion of the shaft, said balloon covering said electrode; and
a radiotherapeutic means for conducting radiotherapy on the affected part said radiotherapeutic means comprising at least one radiation source passage (191,116,405) as a part of said shaft, said at least one radiation source passage extending parallel to but off the axis of the shaft, and at least one radiation source guiding tube (185,408) with a radiation source inserted therein, said at least one radiation source guiding tube being insertable in said at least one radiation source passage,
so that thermotherapy and radiotherapy can be conducted at the same time,
wherein
said intra-cavity thermotherapeutic applicator comprises at least one mark (201,211,212,231,432) to enable adjustment of said at least one radiation source passage to oppose the affected part by rotation of the flexible shaft about its axis.

2. The medical apparatus according to claim 1, wherein said at least one mark is a X-ray marker (201, 211, 212, 231) provided at the distal end portion of said shaft.

3. The medical apparatus according to claim 2, wherein two X-ray markers are provided on two distal end portions of said shaft wherein the first X-ray marker (211) indicates the position of the distal end of said radiation source passage (116) and the second X-ray maker (212) indicates the position of a portion of said radiation source passage (116) which is proximal with respect to the distal end of said radiation source passage (116).

4. The medical apparatus according to claim 1, wherein said at least one mark (432) is provided at a proximal end portion of said applicator.

5. The apparatus according to any of claims 1 to 4, wherein said thermotherapeutic applicator has a heating section, and means for cooling a surface of the heating section.

6. The apparatus according to claim 5, wherein said thermotherapeutic means further comprises a temperature measuring means provided on a surface of said heating section.

7. The apparatus according to any of claims 1 to 6, wherein said radiation source is iridium, and a high-frequency current of about 13.56 MHz is supplied to said electrode.

8. The apparatus according to any of claims 1 to 7, wherein said balloon is for holding said flexible shaft in place in a body cavity of the patient and for containing contrast medium.

## Patentansprüche

1. Eine medizinische Vorrichtung zur Behandlung eines affizierten Teils eines Patienten, umfassend:
ein thermotherapeutisches Mittel zur Durchführung einer Thermotherapie an dem affizierten Teil, wobei das thermotherapeutische Mittel einen thermotherapeutischen intrakavitären Applikator umfaßt, der einen flexiblen Schaft (115, 403), der eine Achse definiert, und eine Hochfrequenzelektrode (181, 407) und einen Ballon (182, 413), die beide an einem distalen Endabschnitt des Schaftes angebracht sind, wobei der Ballon die Elektrode bedeckt, umfaßt; und
ein radiotherapeutisches Mittel zur Durchführung einer Radiotherapie an dem affizierten Teil, wobei das radiotherapeutische Mittel mindestens einen Strahlungsquellenkanal (191, 116, 405) als einen Teil des Schafts, wobei der mindestens eine Strahlungsquellenkanal sich parallel zu, jedoch neben der Achse des Schaftes erstreckt, und mindestens eine Strahlungsquellenführungsröhre (185, 408) mit einer darin eingesetzten Strahlungsquelle umfaßt, wobei die mindestens eine Strahlungsquellenführungsröhre in den mindestens einen Strahlungsquellenkanal einführbar ist,
so daß Thermotherapie und Radiotherapie zur gleichen Zeit durchgeführt werden können,
wobei
der thermotherapeutische intrakavitäre Applikator mindestens eine Markierung (201, 211, 212, 231, 432) umfaßt, um eine Justierung des mindestens einen Strahlungsquellenkanals zu ermöglichen, so daß dieser durch Drehung des flexiblen Schafts um seine Achse gegenüber dem affizierten Teil angeordnet ist.

2. Die medizinische Vorrichtung nach Anspruch 1, wobei die mindestens eine Markierung eine Röntgenmarkierung (201, 211, 212, 231) ist, die an dem distalen Endabschnitt des Schafts vorgesehen ist.

3. Die medizinische Vorrichtung nach Anspruch 2, wobei zwei Röntgenmarker an zwei distalen Endabschnitten des Schaftes vorgesehen sind, wobei der erste Röntgenmarker (211) die Position des distalen Endes des Strahlungsquellenkanals (116) anzeigt und der zweite Röntgenmarker (212) die Position eines Abschnitts des Strahlungsquellenkanals (116), der in Bezug auf das distale Ende des Strahlungsquellenkanals (116) proximal ist, anzeigt.

4. Die medizinische Vorrichtung nach Anspruch 1, wobei die mindestens eine Markierung (432) an einem proximalen Endabschnitt des Applikators vorgesehen ist.

5. Die Vorrichtung nach einem der Ansprüche 1 bis 4, wobei der thermotherapeutische Applikator einen Heizabschnitt und Mittel zur Kühlung einer Oberfläche des Heizabschnitts aufweist.

6. Die Vorrichtung nach Anspruch 5, wobei das thermotherapeutische Mittel ferner ein Temperaturmeßmittel umfaßt, das an einer Oberfläche des Heizabschnitts vorgesehen ist.

7. Die Vorrichtung nach einem der Ansprüche 1 bis 6, wobei die Strahlungsquelle Iridium ist und der Elektrode ein Hochfrequenzstrom von ungefähr 13,56 MHz zugeführt wird.

8. Die Vorrichtung nach einem der Ansprüche 1 bis 7, wobei der Ballon zum Halten des flexiblen Schafts an einer Stelle in einem Körperhohlraum des Patienten und zum Enthalten von Kontrastmedium dient.

## Revendications

1. Appareil médical pour traiter une partie affectée d'un patient, comprenant :
un moyen thermothérapeutique servant à effectuer une thermothérapie sur la partie affectée, ledit moyen thermothérapeutique comprenant un applicateur thermothérapeutique intracavité possédant une tige souple (115, 403) définissant un axe, et une électrode à haute fréquence (181, 407) et un ballonnet (182, 413) montés tous les deux sur une partie d'extrémité distale de la tige, ledit ballonnet couvrant ladite électrode ; et
un moyen radiothérapeutique servant à effectuer une radiothérapie sur la partie affectée, ledit moyen radiothérapeutique comprenant au moins un passage de source de rayonnement (191, 116, 405) en tant que partie de ladite tige, ledit au moins un passage de source de rayonnement s'étendant parallèlement à, mais hors de, l'axe de la tige, et au moins un tube de guidage de source de rayonnement (185, 408), une source de rayonnement y étant introduite, ledit au moins un tube de guidage de source de rayonnement pouvant être introduit dans ledit au moins un passage de source de rayonnement,
de façon à pouvoir effectuer simultanément une thermothérapie et une radiothérapie,
dans lequel
ledit applicateur thermothérapeutique intracavité comprend au moins un repère (201, 211, 212, 231, 432) permettant un réglage dudit au moins un passage de source de rayonnement pour présentation à la partie affectée par rotation de la tige souple autour de son axe.

2. Appareil médical selon la revendication 1, dans lequel ledit au moins un repère est un marqueur de radiographie (201, 211, 212, 231) disposé au niveau de la partie d'extrémité distale de ladite tige.

3. Appareil médical selon la revendication 2, dans lequel deux marqueurs de radiographie sont disposés sur deux parties d'extrémité distale de ladite tige, dans lesquels le premier marqueur de radiographie (211) indique la position de l'extrémité distale dudit passage de source de rayonnement (116) et le second marqueur de radiographie (212) indique la position d'une partie dudit passage de source de rayonnement (116) qui est proximale par rapport à l'extrémité distale dudit passage de source de rayonnement (116).

4. Appareil médical selon la revendication 1, dans lequel ledit au moins un repère (432) est disposé au niveau d'une partie d'extrémité proximale dudit applicateur.

5. Appareil selon l'une quelconque des revendications 1 à 4, dans lequel ledit applicateur thermothérapeutique comporte une section de chauffe et un moyen servant à refroidir une surface de la section de chauffe.

6. Appareil selon la revendication 5, dans lequel ledit moyen thermothérapeutique comprend en outre un moyen de mesure de température disposé sur une surface de ladite section de chauffe.

7. Appareil selon l'une quelconque des revendications 1 à 6, dans lequel ladite source de rayonnement est de l'iridium, et dans lequel un courant haute fréquence d'environ 13,56 MHz est délivré à ladite électrode.

8. Appareil selon l'une quelconque des revendications 1 à 7, dans lequel ledit ballonnet sert à maintenir ladite tige souple en place dans une cavité corporelle du patient et à contenir un milieu de contraste.
